# EUROPEAN PATENT APPLICATION

(11) **EP 3 782 627 A1**
(43) Date of publication of application: **24.02.2021**
(21) Application number: 19789169.0
(22) Date of filing: 17.04.2019
(51) Int. Cl.: A61K 31/7048, A61P 29/00, C07H 17/08, C07H 1/08, C12P 19/62

(54) **DRUG USED FOR PREVENTING AND/OR TREATING PAIN AND/OR FEVER, COMBINATION PRODUCT, AND USE THEREOF**

(30) Priority: 17.04.2018 CN 201810345619; 17.04.2018 CN 201810345615
(71) Applicant: Shanghai Tonglian Pharmaceutical Co., Ltd., Songjiang District Shanghai 201611 (CN)
(72) Inventor: JIANG, Xunlei, Shenyang, Liaoning 110164 (CN); JIANG, Xundong, Shenyang, Liaoning 110164 (CN)
(74) Representative: Hanna Moore + Curley
(86) International application number: PCT/CN2019/083007
(87) International publication number: WO 2019/201268

(57) **Abstract**

The present disclosure discloses a medicament for preventing and/or treating pain and/or fever, a composite product and use thereof. An effective component of the medicament includes one of carrimycin, isovalerylspiramycin III, isovalerylspiramycin II and isovalerylspiramycin I; or a combination of two or three of isovalerylspiramycin I, isovalerylspiramycin II and isovalerylspiramycin III.

## Description

### TECHNIAL FIELD

The present disclosure belongs to the field of medicament application, and in particular relates to a medicament for preventing and/or treating pain and/or fever, a composite product and use thereof.

### BACKGROUND

Pain is defined by the International Pain Association as an unpleasant feeling and emotional feeling with existing tissue damages or with potential tissue damages. In 1995, the American Pain Society put forward that pain was the fifth vital sign, and became an important indicator of vital signs together with the blood pressure, body temperature, respiration and pulse.

Cancer pain is one of the most common and unbearable symptoms of cancer patients, and it is also one of the most difficult symptoms to control. About 30%-50% of cancer patients have different degrees of pains, and 60%-90% of advanced cancer patients have moderate or severe pains, which seriously affect the quality of life of cancer patients.

In order to improve the quality of life of patients with long-term pains, scholars all over the world are trying to find new methods to treat pains, in order to save patients from pains. In view of the above-mentioned diseases, although there are many drugs used to treat pains at present, most of them have slow efficacy, long treatment period, great side effects and strong dependence, and are limited by patients' tolerance, resulting in poor analgesic effects for some patients.

Carrimycin, also known as Bitespiramycin and Shengjimycin, is a new type of antibiotic with 4"-isovalerylspiramycin as a main component, formed by cloning the 4"-isovaleryl transferase gene (4"-o-acyl-transferase) of carbomycin-producing bacteria into spiramycin producing bacteria through transgenic technology, directionally acylating spiramycin 4"-OH, and adding an isovaleryl side chain at the 4" position under the collaboration between the Institute of Biotechnology of the Chinese Academy of Medical Sciences and the applicant.

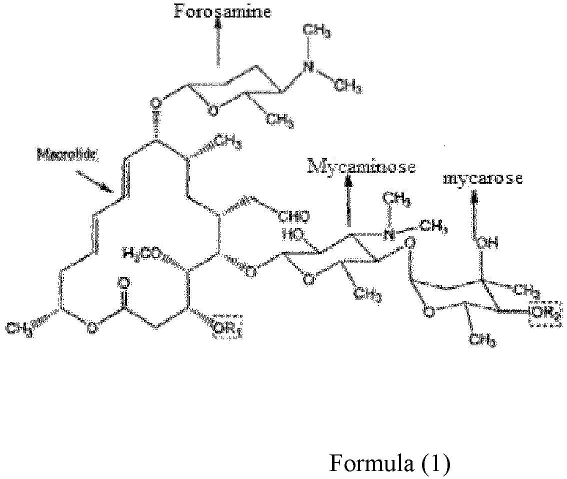

Carrimycin is composed of a variety of spiramycin derivatives, with the total content of isovalerylspiramycins (I+II+III), the main active ingredient, not less than 60% and the total content of acylated spiramycin not less than 80%, and it is an acceptable pharmaceutical composition in pharmacy. The central structure of the carrimycin is a 16-membered lactone ring, which is connected with a molecule of forosamine, a molecule of mycaminose, and a molecule of mycarose. Its main components, isovalerylspiramycins I, II, III, structurally differ from spiramycin in that the group attached to the 4" position of mycarose is isovaleryl instead of hydroxyl. The chemical structure of the carrimycin, as shown in a formula (1), contains more than ten kinds of components. At present, the composition standard of the finished product of carrimycin is that isovalerylspiramycin III is ≥ 30%, the total ratio of isovalerylspiramycin I, II, III is ≥ 60%, the proportion of total acylated spiramycin is ≥ 80%, and the sum of other unknown components is ≤ 5%.

Carrimycin is a 16-membered macrolide antibiotic with active groups of carboxyl, alkoxy, epoxy, ketone and aldehyde groups and a pair of conjugated C=C, with molecular weight of about 884-982. Due to the similar chemical structure, carrimycin and macrolide antibiotics have a lot in common: they are easily soluble in most organic solvents such as esters, acetone, chloroform, and alcohols, and are slightly soluble in petroleum ether, and insoluble in water; their molecular structures contain two dimethylamine groups and is weakly alkaline, and thus they are easily soluble in acidic aqueous solutions; they have a "negative solubility" quality that decreases in solubility with the increasing temperature. Because the main component of carrimycin, isovalerylspiramycin, has a longer carbon chain at the 4" position, it has a poor hydrophilicity, and its solubility in water is less than that of spiramycin and 4"-acetylspiramycin.

Carrimycin is a white non-crystalline powder with a slight hygroscopicity, a specific rotation of about -80.8°, and a maximum ultraviolet absorption wavelength of 231 to 232nm. It has a weak fluorescent chromophore itself, and reacts to appear purple so as to produce strong purple fluorescence when encountered with concentrated sulfuric acid or hydrochloric acid, with the maximum absorbance at 231-232nm.

This drug has good lipophilicity, strong tissue penetration ability, fast oral absorption, long-term maintenance in the body, and sustained post-antibiotic effect. According to the relationship between the efficacy and the chemical conformation, after the acylation of the macrolide antibiotic at the 4" position, its lipophilicity and in vivo activity are improved, the in vivo antibacterial activity and clinical treatment effect have been significantly improved, and the stability of the antibiotic in the body is also enhanced with the growth of the carbon chain of the 4"-hydroxy ester, i.e., isovalerylspiramycin> butyrylspiramycin> propionylspiramycin> acetylspiramycin.

Preliminary in vivo and in vitro pharmacodynamic tests show that the drug not only has good antibacterial activity on most G⁺ bacteria, but also has a certain effect on some G⁻ bacteria, and various technical indexes are obviously superior to azithromycin, erythromycin, acetylspiramycin and midecamycin, especially has the strongest antibacterial activity on mycoplasma pneumoniae, and has certain antibacterial activity on erythromycin resistant bacteria, neisseria gonorrhoeae, pneumococcus, staphylococcus aureus, pesudomonas pyocyaneum, himophilus influenzae, haemophilus influenzae, bacteroides fragilis, legionella, bacillus multiforme and clostridium perfringens, and has little cross resistance to erythromycin resistant staphylococcus aureus clinically. Carrimycin will be mainly used to treat Gram-positive bacteria infectious diseases, especially upper respiratory infection, and may be used for urinary system infection, etc.

Pharmacokinetic research results show that the active effective components in carrimycin are mainly isovalerylspiramycins I, II and III. Carrimycin is rapidly metabolized into spiramycin after entering the body, and its oral absolute bioavailability is 91.6% on average based on the AUC₀₋ₜ sum of the parent drugs isovalerylspiramycins I, II, III and the active metabolites spiramycins I, II and III. Literature reports that the absolute oral bioavailability of spiramycin is 30-40%. This indicates that the structure of isovalerylspiramycin obviously improves the bioavailability of spiramycin, the active ingredient. The elimination of carrimycin is slower after a single dose, and T_{1/2β} is between 23 and 27 hours.

In view of that, the present disclosure has been proposed.

### SUMMARY

The technical problem to be solved by the disclosure is to overcome the defects of the prior art and the disclosure provides a medicament for preventing and/or treating pain and/or fever, a composite product and use thereof.

To solve the above technical problems, the basic idea of the technical solution adopted by the present disclosure is as follows:
The present disclosure is to provide a medicament for preventing and/or treating pain and/or fever, an effective component of the medicament comprises one of carrimycin, isovalerylspiramycin III, isovalerylspiramycin II and isovalerylspiramycin I;
or a combination of two or three of isovalerylspiramycin I, isovalerylspiramycin II and isovalerylspiramycin III.

Furthermore, the medicament comprises a pharmaceutically acceptable carrier, and the medicament is prepared into pharmaceutically acceptable tablets, capsules, pills, injections, sustained-release agents and various microparticle delivery systems.

Furthermore, a dosage of the medicament is in a range from 10 to 80 mg/kg.

The present disclosure further provides a medicament for preventing and/or treating pain and/or fever, an effective component of the medicament comprises at least one selected from a group consisting of:
a derivative, a pharmaceutically acceptable salt, a solvate, a metabolite, a stereoisomer, a tautomer, a polymorph and a drug precursor of carrimycin;
a derivative, a pharmaceutically acceptable salt, a solvate, a metabolite, a stereoisomer, a tautomer, a polymorph and a drug precursor of isovalerylspiramycin III;
a derivative, a pharmaceutically acceptable salt, a solvate, a metabolite, a stereoisomer, a tautomer, a polymorph and a drug precursor of isovalerylspiramycin II; and
a derivative, a pharmaceutically acceptable salt, a solvate, a metabolite, a stereoisomer, a tautomer, a polymorph and a drug precursor of isovalerylspiramycin I.

Furthermore, the medicament comprises a pharmaceutically acceptable carrier, and the medicament is prepared into pharmaceutically acceptable tablets, capsules, pills, injections, sustained-release agents and various microparticle delivery systems.

Furthermore, a dosage of the medicament is in a range from 10 to 80 mg/kg.

The present disclosure further provides a composite product for preventing and/or treating pain and/or fever, the composite product comprises a first drug, and an effective component of the first drug includes one of carrimycin, isovalerylspiramycin III, isovalerylspiramycin II and isovalerylspiramycin I;
or a combination of two or three of isovalerylspiramycin I, isovalerylspiramycin II and isovalerylspiramycin III.

Furthermore, the composite product further comprises a second drug, and the second drug includes at least one of related medicaments for preventing and/or treating pain and/or fever;
preferably, the related medicaments for preventing and/or treating pain and/or fever include narcotic analgesics, antipyretic analgesics, antibiotics and antiviral drugs.

The present disclosure further provides a composite product for preventing and/or treating pain and/or fever, the composite product includes a first drug, an effective component of the first drug comprises at least one selected from a group consisting of:
a derivative, a pharmaceutically acceptable salt, a solvate, a metabolite, a stereoisomer, a tautomer, a polymorph and a drug precursor of carrimycin;
a derivative, a pharmaceutically acceptable salt, a solvate, a metabolite, a stereoisomer, a tautomer, a polymorph and a drug precursor of isovalerylspiramycin III;
a derivative, a pharmaceutically acceptable salt, a solvate, a metabolite, a stereoisomer, a tautomer, a polymorph and a drug precursor of isovalerylspiramycin II;
a derivative, a pharmaceutically acceptable salt, a solvate, a metabolite, a stereoisomer, a tautomer, a polymorph and a drug precursor of isovalerylspiramycin I.

Furthermore, the composite product further comprises a second drug, and the second drug comprises at least one of related medicaments for preventing and/or treating pain and/or fever; the related medicaments for preventing and/or treating pain and/or fever include narcotic analgesics, antipyretic analgesics, antibiotics and antiviral drugs.

The present disclosure further provides use of any one medicament as above or any one composite product as above in preventing and/or treating pain and/or fever.

Furthermore, the pain includes cancer pains. The pain includes nociceptive pains and neuropathic pains.

The present disclosure further provides use of any one medicament as above or any one composite product as above in reducing or preventing synthesis of a prostaglandin, inhibiting release of a substance P, inhibiting reuptake of a serotonin and a norepinephrine, and inhibiting the activity of a cyclooxygenase.

Endogenous pain-causing factors known to cause pains include the prostaglandin, the substance P, the serotonin and the norepinephrine. The mechanism of fever is that pathogens stimulate macrophages to release cytokines, which causes epoxymycin to catalyze arachidonic acid to produce prostaglandins, thus causing the fever, pain and inflammation.

The carrimycin of the present disclosure can be separated and prepared according to the method of the prior art, for example, prepared according to the method of Example 1 of Chinese Patent Application with the application number of CN101785778A.

After adopting the above technical solution, compared with the prior art, the present disclosure has the following beneficial effects:
The medicament and the composite product of the disclosure have a better treatment effect on pain and/or fever, especially have an obvious analgesic effect on cancer pain, and have the advantages of fast drug effects, a short treatment period, small side effects, weak dependence, no limitation of patient tolerance, and important economic and social benefits.

The specific embodiments of the present disclosure will be described in further detail with reference to the accompanying drawings.

### BRIEF DESCRIPTION OF THE DRAWINGS

The attached drawings, as a part of the present disclosure, are used to provide a further understanding of the present disclosure. The illustrative embodiments of the present disclosure and their descriptions are used to explain the present disclosure, but do not constitute an improper limitation of the present disclosure. Obviously, the drawings in the following description are only some examples, and for those of ordinary skill in the art, other drawings can be obtained according to these drawings without paying creative work. In the drawings:
Fig. 1 is the mechanical pain threshold of the inoculated hind limbs of inoculation sides of rats according to the present disclosure.
Fig. 2 is the mechanical pain threshold of the hind limb opposite to the inoculation side of the rat of the present disclosure.
Fig. 3 is the hot pain threshold of the hind limb of the inoculation side of the rat according to the present disclosure.
Fig. 4 is the hot pain threshold of the hind limb opposite to the inoculation side of the rat of the present disclosure.

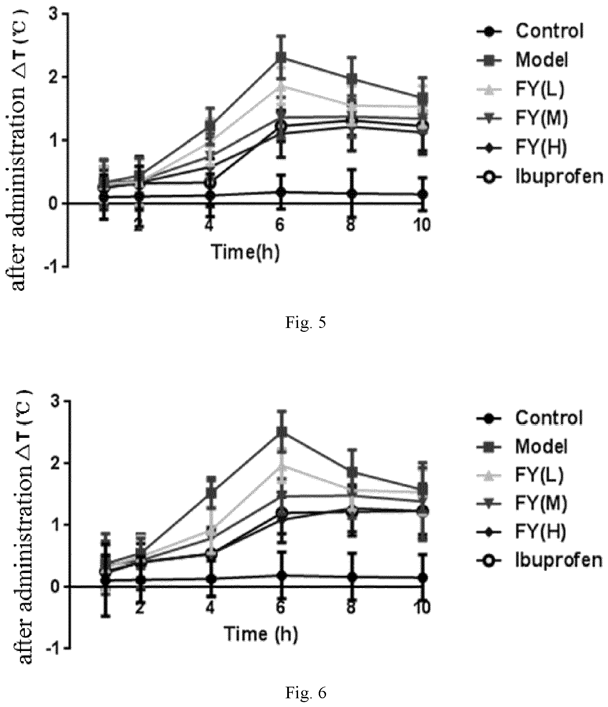

Fig. 5 is a line chart of the changes of the body temperature of rats of a LPS fever model caused by the medicament and composite product of the present disclosure.
Fig. 6 is a line chart showing the changes of the body temperature of rats of a dry yeast fever model caused by the medicament and composite product of the present disclosure.

It should be noted that these drawings and written descriptions are not intended to limit the conceptual scope of the present disclosure in any way, but to explain the concept of the present disclosure to those skilled in the art by referring to specific embodiments.

### Specific Embodiments

In order to make the objectives, technical solutions and advantages of the embodiments of the present disclosure clearer, the technical solutions in the embodiments will be described clearly and completely with reference to the drawings in the embodiments of the present disclosure. The following embodiments are used to illustrate the present disclosure, but are not intended to limit the scope of the present disclosure.

### Example 1: coated tablets

| | |
|---|---|
| Specification: 200mg/350mg | |
| Prescription of the tablet core: | |
| Medicament A | 200g |
| microcrystalline cellulose | 110g |
| sodium starch glycolate | 22g |
| povidone K₃₀ (5%) | 15g |
| magnesium stearate | 3g |
| | formulated into 1000 tablets |
| Prescription of the coating solution: | |
| Opadry II | 21g |
| Distilled water | proper amount |
| | formulated into 105mL |

The medicament A is one of carrimycin, isovalerylspiramycin III, isovalerylspiramycin II and isovalerylspiramycin I; or a combination of two or three of isovalerylspiramycin I, isovalerylspiramycin II and isovalerylspiramycin III.

### The preparation process:

Preparation of the tablet core: the main drug and the excipients respectively passed through a 100-mesh sieve, and a prescription dosages of raw powder of medicament A and microcrystalline cellulose with a 1/2 prescription dosage of sodium starch glycolate were uniformly mixed, then an aqueous solution of 5% povidone K₃₀ was added to prepare a soft material. An 18-mesh sieve was used for granulating, and the wet granules were dried under a ventilated condition at 60DEG Cfor 2h. After the wet granules were dried, a 18-mesh screen was used for dispersing the granules, then a 1/2 prescription dosage of sodium starch glycolate and magnesium stearate were added. And after the materials were uniformly mixed, the mixture was tabletted by using a shallow concave die of a diameter of 11mm, to obtain a drug-containing tablet core with the tablet weight of 350mg and the hardness of 6.5kg.

Preparation of the coating solution: the required amount of Opadry II (white color) was weighed, the required amount of water was added into the preparation container in batches, after all of the water has been added, the stirring speed was reduced to make the spiral disappear, and the stirring was continued to be performed for 30min to obtain the coating solution.

Preparation of the film coated tablet: the tablet core was placed into a coating pan, the coating conditions were determined, and coating was carried out with the main rotation speed of 20r/min, the air inlet temperature of 40DEG C, the air outlet temperature of 30DEG C, the atomization pressure of 0.02Mpa and the spraying flow rate of 1ml/min. And after a constant state was achieved, the coating was continuously to be sprayed for 1.5h to obtain a tablet with a smooth surface and a uniform colour and lustre. The tablet were qualified if it were in compliance with the inspecting standards of thin-film coating. The coating adds the weight by approximately 5%.

### Example 2: plain tablets (calculated for 10000 tablets)

### Prescription:

| | |
|---|---|
| medicament A | 1000g |
| low-substituted hydroxypropyl cellulose (5%) | 92.5g |
| sodium starch glycolate (3%) | 55.5g |
| magnesium stearate (1%) | 18.5g |
| starch | the total weight subtracts the weights of the other raw materials and excipients |
| total weight | 1850g |

The medicament A is one of carrimycin, isovalerylspiramycin III, isovalerylspiramycin II and isovalerylspiramycin I; or a combination of two or three of isovalerylspiramycin I, isovalerylspiramycin II and isovalerylspiramycin III.

The preparation process: a proper amount of starch was weighed, diluted to a concentration of 15%, and heated to be a paste, to obtain an adhesive the main material, row powder of medicament A, and the excipients starch, low-substituted hydroxypropyl cellulose, sodium starch glycolate and magnesium stearate passed through a 100-mesh sieve, respectively; and prescription dosages of the main material and the excipients were weighed. After the raw powder of medicament A, starch and low-substituted hydroxypropyl cellulose were fully and uniformly mixed, the starch paste with the starch concentration of 15% was used to prepare the mixture into a soft material which was granulated by a 14-mesh sieve, and granules were dried at 50-60DEG C to control the moisture content at 3-5%. A 14-mesh sieve was used for dispersing the granules, and then sodium carboxymethyl starch and magnesium stearate were added to be mixed, and the granule content was measured. The weight of the tablet was calculated according to the granule content, and the mixture was tabletted (with a Φ9 mm shallow concave punch), then the difference in the weight of the tablets was detected. After passing the test, the tablets were packaged.

### Example 3: capsules (calculated for 10000 granules)

### Prescription:

| | |
|---|---|
| medicament A | 1000g |
| starch | 1080 subtracts the weight of medicament A |
| medicinal No. 3 capsule | 1000 granules |
| liquid paraffin | 50ml |

The medicament A is one of carrimycin, isovalerylspiramycin III, isovalerylspiramycin II and isovalerylspiramycin I; or a combination of two or three of isovalerylspiramycin I, isovalerylspiramycin II and isovalerylspiramycin III.

The preparation process: the main material, raw powder of medicament A, and the excipient medicinal starch were separately weighed according to the dosages of the process prescription, and then fully mixed infor 1.5-2 hours. The data obtained by sampling and detecting the content should be substantially consistent with the theoretical data (the weight contained by each of the capsules was approximately 0.105g); and the qualified No. 3 medicinal capsule and the mixed raw materials to be loaded were filled in a filling device according to the operation requirements of an automatic capsule machine, and the filled capsules were subjected to a difference test (±10% or less, <0.3g) to see if the dissolution rate meets the requirements or not. The capsules that meet the requirements after being tested were put into a polishing machine to be polished for 15-20 minutes with the liquid paraffin added, and then were taken out to be tested by finished product packaging boxes.

### Example 4: dried syrup (calculated for 10000 bags)

### Prescription:

| | |
|---|---|
| medicament A | 1250g |
| citric acid (0.5%) | 15g |
| sucrose | the total weight subtracts the weights of the other raw materials and excipients |
| total weight, approximately | 500g |
| pigment (Curcumin) | approximately 1g |

The medicament A is one of carrimycin, isovalerylspiramycin III, isovalerylspiramycin II and isovalerylspiramycin I; or a combination of two or three of isovalerylspiramycin I, isovalerylspiramycin II and isovalerylspiramycin III.

The preparation process: the raw powder of medicament A, citric acid and sucrose were respectively grinded into granules by using a jet-stream pulverizer, and 85% of the granules passed through a 300-mesh sieve, 15% of the granules passed through a 180-mesh sieve. Then the fine powder after grinding was weighed according to the prescription amount and fully mixed for for 1-1.5 hours, the content was measured, the loading capacity was calculated(the theoretical filling amount was 500mg per bag). Then the mixture was put into a bagging machine, aluminum foil paper was installed, and filling was carried out according to the operation requirements of a filling machine. The difference was allowed to be within ±5 %, and after the filling, the outer packaging was carried out after passing the inspection.

### Example 5: granule preparation (calculated for 10000 bags)

### Prescription:

| | |
|---|---|
| medicament A | 1250g |
| sugar powder | 20000g |
| dextrin | 9000g |
| 5% PVP-K₃₀ | proper amount |

The medicament A is one of carrimycin, isovalerylspiramycin III, isovalerylspiramycin II and isovalerylspiramycin I; or a combination of two or three of isovalerylspiramycin I, isovalerylspiramycin II and isovalerylspiramycin III.

The preparation process: the raw powder of medicament A, sugar powder and dextrin passed through a 120-mesh sieve, and the raw powder of medicament A, sugar powder and dextrin were weighed according to the prescription amount and uniformly mixed. And the above uniformly mixed materials were made into a soft material with a 5% PVP-K₃₀ mucilage, and then the soft material was granulated with a swinging granulation machine, dried at 70DEG C and subjected to granule dispersion, and the resulting granules were subpackaged after being qualified for inspection.

### Example 6: freeze-dried powder injection

The process: 500mg of raw powder of medicament A was uniformly mixed with an equal molar amount of hexanedioic acid, and the mixture was dissolved in 5 ml of water to obtain a faint yellow clear solution having a pH between 4.6 and 5.6. Further, 40 mg of mannitol was added as a lyophilized proppant into the faint yellow clear solution, and after being frozen rapidly at a low temperature for 9 hours, the material was freeze-dried to obtain a faint yellow loose mass, which was dissolved in 10 ml of sterile water before being used.

The medicament A is one of carrimycin, isovalerylspiramycin III, isovalerylspiramycin II and isovalerylspiramycin I; or a combination of two or three of isovalerylspiramycin I, isovalerylspiramycin II and isovalerylspiramycin III.

### Experimental example 1. Establishment of tibia cancer pain model

Walker rat breast cancer cells were purchased from Shanghai Institute of Biomedical Engineering. The abdominal cavity of female Wistar rats of 70-80g was inoculated with 1ml of ascites tumor (about 2×107 cells /ml). After 7-10 days, 5ml of ascites was extracted and centrifuged for 3 minutes (1300rpm). After precipitation, washing was performed with 10ml PBS, and then centrifuged and precipitated again. Then, resuspending was performed with PBS, counting was performed, the cell concentration was adjusted to 1×10⁷ cells /ml by PBS and placed in an ice box for later use.

Rats were anesthetized by intraperitoneal injection of 10% chlorate hydrate (4ml/kg body weight), a skin was prepared at the right knee joint, and the skin was disinfected with 75% alcohol. The knee joint was fixed with the left hand to make the skin on its surface tense. A hole with a depth about 1 cm was drilled along the longitudinal axis of tibia at the medial edge of patellar ligament of the knee joint with a 7 # needle. Then, a microsampler with tumor cells (before injection, 1µl of air, 2µl of gelatin sponge aqueous solution, 1µl of air, 4µl of tumor cells or PBS were inhaled in turn) was used, and 4µl of tumor cells was injected into the tibia bone marrow cavity; The rats in the control group were injected with the same amount of PBS, and finally all of them were sealed with 2µl of gel sponge solution. After injection, the needles were kept for 1 minute to prevent the injection from oozing out.

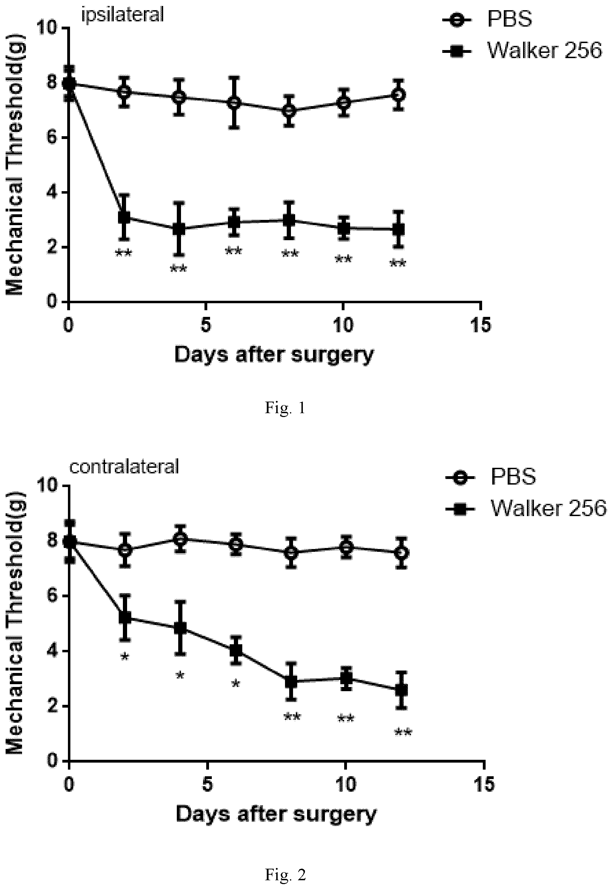

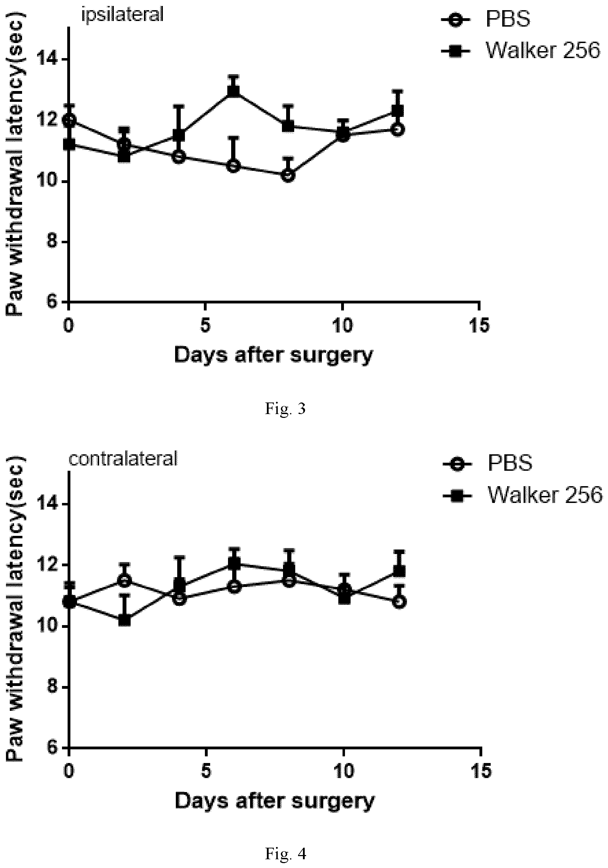

Rats were divided into two groups: a PBS injection control group and a Walker256 model group. The radiation paw withdrawal latency (PWL) was measured before modeling as the basic pain threshold, and the PWL was measured every other day after modeling as the thermal hyperalgesia threshold. See Table 2 for details, and the corresponding graphs are shown in Figures 3 and 4. At the same time, the mechanical pain caused by von Frey filament was measured. The threshold of mechanical pain was measured before modeling as the basic pain threshold, and the threshold of mechanical allodynia was measured every other day after modeling. See Table 1 for details, and the corresponding graphs are shown in Figs. 1 and 2.

**Table 1. van Frey mechanical pain threshold of rats after modeling (g) (x±s, n=8)**

| group | | time (day) | | | | | | |
|---|---|---|---|---|---|---|---|---|
| | | 0 | 2 | 4 | 6 | 8 | 10 | 12 |
| same side | PBS | 8.0±0.5 | 7.7±0.5 | 7.5±0.6 | 7.3±0.9 | 7.0±0.5 | 7.3±0.5 | 7.6±0.6 |
| | Walker256 | 8.0±0.6 | 3.1±0.8** | 2.7±0.9** | 2.9±0.5** | 3.0±0.6** | 2.7±0.4** | 2.7±0.6** |
| oposite side | PBS | 8.0±0.7 | 7.7±0.6 | 8.1±0.5 | 7.9±0.4 | 7.6±0.5 | 7.8±0.4 | 7.6±0.5 |
| | Walker256 | 8.0±0.6 | 5.2±0.8* | 4.9±0.9* | 4.0±0.5** | 2.9±0.6** | 3.0±0.4** | 2.6±0.6** |

| | | | | | | | | |
|---|---|---|---|---|---|---|---|---|
| * *p*<0.05 as compared with the control group, ** *p*<0.01 as compared with the control group | | | | | | | | |

**Table 2. Hot pain threshold of rats after modeling (sec) (x̅±s, n=8)**

| **group** | | **time** (day) | | | | | | |
|---|---|---|---|---|---|---|---|---|
| | | 0 | 2 | 4 | 6 | 8 | 10 | 12 |
| **same side** | PBS | 12.0±0.5 | 11.2±0.5 | 10.8±0.6 | 10.5±0.9 | 10.2±0.5 | 11.5±0.5 | 11.7±0.6 |
| | Walker256 | 11.2±0.6 | 10.8±0.8 | 11.5±0.9 | 12.9±0.5 | 11.8±0.6 | 11.6±0.4 | 12.3±0.6 |
| **oposite side** | PBS | 10.8±0.5 | 11.5±0.5 | 10.9±0.6 | 11.3±0.9 | 11.5±0.5 | 11.2±0.5 | 10.8±0.4 |
| | Walker256 | 10.8±0.6 | 10.2±0.8 | 11.3±0.8 | 12.0±0.5 | 11.8±0.7 | 10.9±0.4 | 11.8±0.6 |

The results showed that, compared with rats injected with the PBS, the threshold of mechanical pain caused by Von Frey filament in the hind limb of the inoculation side decreased significantly from the second day after Walker256 was inoculated (P<0.05). Similar to the inoculation side, the mechanical pain threshold of the opposite hind limb of rats inoculated with Walker256 changed similarly. However, in the experiment of heat-sensitive pain, it was found that there was no significant difference in thermal hyperalgesia threshold between the hind limbs of rats inoculated with Walker256 (P<0.05).

### Experimental example 2. Effect of medicament A on mechanical pain threshold in rats with tibial cancer pain

The rats were randomly divided into 6 groups: a model group, administration groups with 10, 20, 40 and 80mg/kg of medicament A, and a positive drug (morphine hydrochloride) control group. After successfully establishing the model of tibia cancer pain, continuous intragastric administration was performed for 12 days. The rats in the model group were intragastrically given the same volume of normal saline. And the positive drug group was given 15mg/kg of morphine hydrochloride on the last day of the experiment. The mechanical pain caused by a van Frey fine needle was used as an index to measure the change of mechanical pain threshold in rats, see Table 3 for details. The effective components of medicament A include one of carrimycin, isovalerylspiramycin III, isovalerylspiramycin II and isovalerylspiramycin I; or a combination of two or three of isovalerylspiramycin I, isovalerylspiramycin II and isovalerylspiramycin III.

**Table 3. van Frey mechanical pain threshold of rats after administration (g) (x̅±s, n=8)**

| group | administration (mg/kg ) dosage | same side | analgesic (%) rate | opposite side | analgesic (%) rate |
|---|---|---|---|---|---|
| model control | 0 | 1.8±0.3 | -- | 2.7±0.5 | -- |
| medicament A | 10 | 1.9±0.2 | 5.5 | 2.9±0.4 | 7.4 |
| | 20 | 3.3±0.4** | 83.3 | 3.8±0.6** | 40.7 |
| | 40 | 4.1±0.3*** | 127.8 | 5.1±0.3*** | 88.9 |
| | 80 | 5.2±0.6*** | 188.9 | 6.1±0.2*** | 125.9 |
| positive control | 15 | 5.5±0.2*** | 205.5 | 6.3±0.5*** | 133.3 |

| | | | | | |
|---|---|---|---|---|---|
| ** *p*<0.01 as compared with the control group, *** *p*<0.001 as compared with the control group | | | | | |

The results showed that, compared with the model control group, the mechanical pain threshold of modeled hind limbs of rats in the administration groups of 20, 40, 80 mg/kg of medicament A and positive drug control group increased significantly (p<0.01), and the analgesic rates reached 83.3%, 127.8%, 188.9% and 205.5%, respectively. The effects of drug modeling on the mechanical pain threshold of hind limbs at the opposite sides were similar. The analgesic rates were 40.7%, 88.9%, 125.9% and 133.3%, respectively. The above results indicated that the medicament A could significantly increase the mechanical pain threshold of rats suffering from cancer pain.

To sum up, a rat model of tibia cancer pain was established, and Walker256 breast cancer cells were injected into the tibia bone marrow cavity of rats by a micro-sampler. With the growth of tumor cells, there was no obvious thermal hyperalgesia in the hind limbs of rats, but obvious mechanical allodynia occurred, suggesting that the model was successfully established. After the establishment of the model, the mechanical pain threshold of bilateral hind limbs of rats was tested after 12 days of continuous intragastric administration. The results showed that the mechanical pain threshold of bilateral hind limbs of rats in the administration group with 20-80 mg/kg of medicament A increased significantly. Administration of 20-80 mg/kg of medicament A significantly increased the mechanical pain threshold of rats with cancer pains. The experimental results showed that the medicament A could be used to treat cancer pains.

### Experimental example 3. Analgesic experiment of writhing method in mice

The mouse writhing method is a classic method for screening analgesics. Its principle is to observe the tolerance degree of chemical pain in administrated mice. The method is to inject 1.44% acetic acid into the abdominal cavity of the administrated mice at 0.2ml/mouse, and record the times of animal writhing within 30 minutes. Writhing index is that the abdomen crawls forward against the bottom of the cylinder, and the hind legs are straightened or the hips are raised and twisted to one side, so the sensitivity of writhing experiment is high.

Sixty healthy Kunming mice, male, with a weight of 18-22g, were randomly divided into 6 groups after passing the quarantine inspection, namely a blank control group, medicament A groups with dosages of 10 mg/kg, 20 mg/kg, 40 mg/kg, 80 mg/kg, and a positive drug control group with 15 mg/kg of morphine hydrochloride, with 10 mice in each group. Before the experiment, mice in each group were given intragastric administration, while mice in the blank control group were given the same volume of normal saline every day for 3 consecutive days. Morphine hydrochloride, a positive drug, was given only once on the day of the experiment. The effective components of medicament A include one of carrimycin, isovalerylspiramycin III, isovalerylspiramycin II and isovalerylspiramycin 1; or a combination of two or three of isovalerylspiramycin I, isovalerylspiramycin II and isovalerylspiramycin III.

One hour after administration, each mouse was injected with 0.2ml/20g of 1.44% glacial acetic acid intraperitoneally. After injection of acetic acid for 20 minutes, the writhing times of each mouse were recorded, and the differences between groups were compared. Then, the analgesic percentage of the medicament was calculated, analgesic rate % = (writhing times of the blank group-writhing times of the administration group) ÷ writhing times of the blank group ×100%, see Table 4 for details.

**Table 4. Times of writhing in mice (x̅±s, n=10)**

| group | dosage of (mg/kg) administration | times of (n) writhing | analgesic (%) rate |
|---|---|---|---|
| blank control | 0 | 55.70±11.17 | - |
| medicament A | 10 | 47.20±4.10 | 15.26 |
| | 20 | 43.18±5.65* | 22.48 |
| | 40 | 35.69±8.31* | 35.92 |
| | 80 | 28.60±5.93** | 48.65 |
| positive control | 15 | 30.17±6.14** | 45.83 |

| | | | |
|---|---|---|---|
| * p<0.05 as compared with the control group, ** p<0.01 as compared with the control group | | | |

The results showed that the number of times of writhing in mice decreased with the increase of the dosage of medicament A, and the analgesic rates of the dosage groups of 10mg/kg, 20mg/kg, 40mg/kg and 80mg/kg of medicament A were 15.26%, 22.48%, 35.92% and 48.65%, respectively. The analgesic rate of the positive drug morphine hydrochloride was 45.83%, therefore, 20-80mg/kg of the medicament A had a significant analgesic effect.

### Experimental example 4. Analgesia experiment on a hot plate in mice

The mouse hot plate method is a classic method for screening analgesics. Its principle is to put the mouse on a hot plate heated to a constant temperature of (55±0.1DEG C) in advance, and when the foot of the mouse is stimulated by the hot plate to produce pain, the time required for foot licking is taken as the pain threshold, which is taken as the observation index, and the changes of threshold before and after medication are compared to evaluate the analgesic effect.

Sixty healthy Kunming female mice, with a weight of 18-22g, were randomly divided into 6 groups after passing the quarantine inspection, namely a blank control group, dosage groups with 10 mg/kg, 20 mg/kg, 40 mg/kg, 80 mg/kg of the medicament A and a positive drug control group with 15 mg/kg of morphine hydrochloride, with 10 mice in each group. Before the experiment, the mice in each group were given intragastric administration. The mice in the blank control group were given the same volume of normal saline every day for 3 consecutive days. The positive drug morphine hydrochloride was given once before the end of the experiment. The effective components of the medicament A include one of carrimycin, isovalerylspiramycin III, isovalerylspiramycin II and isovalerylspiramycin I; or a combination of two or three of isovalerylspiramycin I, isovalerylspiramycin II and isovalerylspiramycin III.

The mice were put on a hot plate pain meter heated to 55DEG C in advance, and the time from putting into the hot plate to licking hind feet was recorded with a stopwatch as the pain threshold of the mice. Before administration, the pain threshold of each mouse was measured, and those who did not exceed 30 seconds were qualified. It was measured once at 30, 60, 90 and 120min after administration. After the end of the experiment, the percentage of pain threshold increase was calculated according to the measured average value of pain threshold. The percentage of pain threshold increase % = (an average pain threshold after administration-an average pain threshold before administration) ÷ the average pain threshold before administration × 100%. See Tables 5 and 6 for details.

**Table 5. Pain threshold of the hot plate method in mice (x̅±s, n=10)**

| group | dosage of administration (mg/kg) | threshold before administration (s) | time to lick hind feet after administration (5) | | | |
|---|---|---|---|---|---|---|
| | | | 30min | 60min | 90mm | 120min |
| blank control | - | 19.47±7.26 | 19.07±6.70 | 20.62±5.17 | 18.19±7.03 | 18.42±6.69 |
| | 10 | 18.69±5.01 | 19.95±6.66 | 21.27±4.57 | 22.17±8.14 | 21.93±5.42 |
| | 20 | 18.37±4.50 | 17.94±8.67 | 20.71±10.48 | 21.10±7.05 | 20.17±4.91 |
| medicament A | 40 | 17.92±5.64 | 21.72±4.14 | 24.33±5.69** | 25.14±8.75** | 20.33±5.16 |
| | 80 | 18.67±5.19 | 27.89±8.54*** | 27.88±9.74*** | 26.58±8.57*** | 21.47±6.17 |
| positive control | 15 | 18.94±6.84 | 31.84±7.17*** | 29.17±6.17*** | 28.77±6.71*** | 26.17±5.14*** |

| | | | | | | |
|---|---|---|---|---|---|---|
| * *p*<0.05 as compared with the control group, ** *p*<0.01 as compared with the control group, *** *p*<0.001 as compared with the control group | | | | | | |

**Table 6. Percentage of pain threshold increase in mice by the hot plate method (n=10)**

| group | dosage of administration (mg/kg) | Percentage of pain threshold increase (%) | | | |
|---|---|---|---|---|---|
| | | 30min | 60min | 90min | 120min |
| blank control | -- | -2.05 | 5.91 | -6.57 | -5.39 |
| medicament A | 10 | 6.74 | 13.80 | 18.62 | 17.34 |
| | 20 A | -2.34 | 12.74 | 14.86 | 9.80 |
| | 40 | 21.21 | 35.77 | 40.29 | 13.45 |
| | 80 | 49.38 | 49.33 | 42.37 | 15.00 |
| positive control | 15 | 68.11 | 54.01 | 51.90 | 38.17 |

The results showed that 30min after administration, the percentage of pain threshold increase of groups of 40mg/kg and 80mg/kg of the medicament A and the positive drug group was 21.21%, 49.38% and 68.11%, respectively, 60min after administration, the percentage of pain threshold increase of groups of 40mg/kg and 80mg/kg of the medicament A and the positive drug group was 35.77%, 49.33% and 54.01%, respectively and 90min after administration, the percentage of pain threshold increase of groups of 40mg/kg and 80mg/kg of the medicament A and the positive drug group was 40.29%, 42.37% and 51.90%, respectively. Therefore, 40-80mg/kg of the medicament A has an obvious analgesic effect.

To sum up, the analgesic experiment of the writhing method in mice showed that the writhing times of mice decreased with the increase of the dosage of the medicament A. And the analgesic rates of the medicament A groups with dosages of 10 mg/kg, 20 mg/kg, 40 mg/kg and 80 mg/kg on pains in mice caused by glacial acetic acid were 15.26%, 22.48%, 35.92% and 48.65%, respectively. The analgesic rate of the positive drug morphine hydrochloride was 45.83%. The analgesic experiment for mice by the hot plate method showed that the time of licking the hind feet of mice was prolonged in different degrees after administration of the medicament A. And the pain threshold of groups with 40mg/kg and 80mg/kg at 30min and 60min was obviously increased. Therefore, the medicament A has an obvious analgesic effect, and has important economic and social benefits. The analgesic experiment of the writhing method in mice showed that the medicament A showed a significant analgesic effect when 20-80 mg/kg is administrated. The analgesic experiment for mice by the hot plate method showed that the medicament A with dosages of 40 mg/kg and 80 mg/kg produced a significant analgesic effect 30min after administration. The experimental results showed that the medicament A could be used for analgesia.

### Experimental example 5. Effect of the medicament A on LPS-induced common fever model in rats

### Experimental animals:

Male healthy Sprague Dawley rats (SPF), with a weight of 220-260 g, purchased from Liaoning Changsheng Biotechnology Co., Ltd., with a license number of SCXK (Liao) 2015-0001.

### Experimental drugs and reagents:

LPS (100mg): (sigma company), batch number: L2880;
Sodium chloride injection (0.45 g, 500 mL): (Shijiazhuang No.4 Pharmaceutical Co., Ltd), Batch number: 101217406; 0.9% sodium chloride injection was dissolved and diluted to 1 mg /ml, then packed in EP tubes and stored at -80DEG C for later use; immediately before use, it was diluted with 0.9% sodium chloride injection into a sterile injection of 10 µg/ml(100 µg/kg).

### Experimental instruments:

Electronic body temperature MC-347: produced by Omron Co., Ltd. (Dalian), with a temperature error of ±0.1DEG C;
TE2101-L electronic balance: Beijing Sartorius Instrument system co., ltd;
PB602-N electronic balance: Mettler-Toledo Instrument co., ltd (Shanghai);
Operation steps are as follows:

### Grouping:

Male SD rats, with a weight of 220-260 g, were fed adaptively for 3 days, ate food and drank water freely with 12 hours of day-night circle. They were randomly divided into 6 groups: a blank group, a model group, groups of the medicament A of 25, 50 and 100mg/kg and a group of positive drug Ibuprofen of 30mg/kg, with 10 rats in each group. Each group was administrated with the drug based on 1ml /100 g body weight by intragastric administration. The normal group and model group were given equal volume of distilled water, the medicament A group and the positive drug group were given corresponding doses of drugs, and each group was given two days continuously. On the third day, after all mice fasted with free access to water for 6 hours, the normal group and the model group were given equal volume of distilled water, and the medicament A group and the positive drug group were given for the last time. After the last administration, except the normal group, rats in other groups were injected with 100 µg /kg of (ip) LPS intraperitoneally and a 20% dry yeast suspension based on 1 ml /100 g subcutaneously in the back, and modeling was performed. At 1 h, 2 h, 4 h, 6 h, 8 h and 10h after administration, anal temperatures were measured according to the above method, and the maximum temperature rise height ΔT was calculated at each time point (ΔT = the body temperature at fever-the basal body temperature).

The experimental results are shown in Table 7:

**Table 7 Effect of the medicament A on the body temperature change of rats of the LPS fever model (means ± s, n = 10)**

| group | dosage (mg/kg) | basal body temperature (°C) | after administration ΔT(°C) | | | | | |
|---|---|---|---|---|---|---|---|---|
| | | | 1h | 2h | 4h | 6h | 8h | 10h |
| normal group | | 36.43 ± 0.35 | 0.11 ± 0.35 | 0.12 ± 0.48 | 0.14 ± 0.34 | 0.19 ± 0.27 | 0.17 ± 0.38 | 0.16 ± 0.26 |
| model group | | 36.90 ± 0.32 | 0.34 ± 0.37 | 0.45 ± 0.28 | 1.23 ± 0.29 | 2.32 ± 0.34 | 1.98 ± 0.34 | 1.68 ± 0.32 |
| | 25 | 36.34 ± 0.48 | 0.32 ± 0.28 | 0.35 ± 0.37 | 0.98 ± 0.37 | 1.87 ± 0.29 | 1.56 ± 0.31 | 1.54 ± 0.33 |
| medicament A | 50 | 36.78 ± 0.36 | 0.33 ± 0.36 | 0.37 ± 0.23 | 0.76 ± 0.35 | 1.37 ± 0.32 | 1.38 ± 0.33 | 1.35 ± 0.22 |
| | 100 | 36.68 ± 0.48 | 0.25 ± 0.34 | 0.34 ± 0.38 | 0.59 ± 0.45 | 1.11 ± 0.37 | 1.22 ± 0.38 | 1.13 ± 0.34 |
| ibuprofen group | 30 | 36.79 ± 0.35 | 0.27 ± 0.27 | 0.33 ± 0.43 | 0.34 ± 0.38 | 1.23 ± 0.24 | 1.32 ± 0.24 | 1.23 ± 0.39 |

According to the above experimental results, it was indicated that compared with the blank group, the fever in the LPS model group was obvious from 2h to 10h, and reached the peak at 6h, and then gradually decreased. Compared with the model group, the medicament A administration group can significantly reduce the body temperature of rats. 50mg/kg and 100mg/kg were significant at 2h and 10h, and each dose group was significant at 4h, 6h and 8h time points. Therefore, it can be concluded that the medicament A can improve the common fever caused by LPS in rats.

### Experimental example 6. Effect of the medicament A on the common fever model of rats induced by dry yeast

Experimental drugs and reagents: in this experiment, the LPS in Experimental example 1 was replaced by dry yeast, which was produced by Hubei Angel Yeast Co., Ltd., and was prepared into a 20% uniform suspension with distilled water of 0-4DEG C before use.

Other experimental drugs and reagents are the same as those in Experimental example 1.

### Experimental instruments: same as Experiment 1.

Operating steps: except that the LPS in Experiment 1 was replaced by the dry yeast, other experimental operating steps and dosage selections were the same as those in Experiment 1. Finally, according to anal temperature measured at 1 h, 2 h, 4 h, 6 h, 8 h and 10h after administration, the maximum body temperature rising height Δ T was calculated at each time point (ΔT = the body temperature at fever-the basal body temperature).

The experimental results are shown in Table 8:

**Table 8 Effect of the medicament A on the body temperature change of rats of the dry yeast fever model (means ± s, n = 10)**

| group | dosage ( mg/kg | basal body temperature (°C) | after administration ΔT(°C) | | | | | |
|---|---|---|---|---|---|---|---|---|
| | | | 1h | 2h | 4h | 6h | 8h | 10h |
| normal group | | 36.78 ± 0.67 | 0.11 ± 0.58 | 0.12 ± 0.37 | 0.14 ± 0.29 | 0.19 ± 0.38 | 0.17 ± 0.38 | 0.16 ± 0.37 |
| model group | | 36.76 ± 0.49 | 0.38 ± 049 | 0.55 ± 0.24 | 1.53 ± 0.25 | 2.52 ± 0.33 | 1.87 ± 0.36 | 1.58 ± 0.36 |
| | 25 | 3.6.54 ± 0.29 | 0.32 ± 0.36 | 0.49± 0.33 | 0.92 ± 0.38 | 1.97 ± 0.28 | 1.57 ± 0.32 | 1.54 ± 0.37 |
| medicament A | 50 | 36.98 ± 0.87 | 0.35 ± 0.39 | 0.43 ± 0.43 | 0.79 ± 0.94 | 1.47 ± 0.29 | 1.48 ± 0.37 | 1.39 ± 0.63 |
| | 100 | 36.38 ± 0.73 | 0.23 ± 0.27 | 0.41 ± 0.45 | 0.54 ± 0.43 | 1.09 ± 0.37 | 1.28 ± 0.38 | 1.23 ± 0.44 |
| ibuprofen group | 30 | 36.49 ± 0.39 | 0.26 ± 0.26 | 0.40 ±0.31 | 0.54 ± 0.38 | 1.20 ± 0.34 | 1.22 ± 0.39 | 1.23 ± 0.38 |

The above experimental results showed that compared with the blank group, the fever in the dry yeast model group was obvious from 2h to 10h, and reached the peak at 6h, and then gradually decreased. Compared with the model group, the medicament A administration group can significantly reduce the body temperature of rats. 50mg/kg and 100mg/kg were significant at 2h and 10h, and each dosage group was significant at 4h, 6h and 8h time points. Therefore, the medicament A can improve the common fever of rats caused by dry yeast.

The above are only preferred embodiments of the present disclosure, and do not limit the present disclosure in any form. Although the present disclosure has been disclosed as preferred embodiments, it is not intended to limit the present disclosure. Without departing from the technical solution of the present disclosure, any person familiar with this patent can make some changes or modifications to equivalent embodiments with equivalent changes by using the technical contents indicated above, but any simple modifications, equivalent changes and modifications made to the above embodiments according to the technical essence of the present disclosure without departing from the technical solution of the present disclosure, still fall within the scope of the present disclosure.

## Claims

1. A medicament for preventing and/or treating pain and/or fever, comprising an effective component, and the effective component of the medicament comprises one of carrimycin, isovalerylspiramycin III, isovalerylspiramycin II and isovalerylspiramycin I;
or a combination of two or three of isovalerylspiramycin I, isovalerylspiramycin II and isovalerylspiramycin III.

2. The medicament for preventing and/or treating pain and/or fever according to claim 1, wherein the medicament comprises a pharmaceutically acceptable carrier, and the medicament is prepared into pharmaceutically acceptable tablets, capsules, pills, injections, sustained-release agents and various microparticle delivery systems.

3. The medicament for preventing and/or treating pain and/or fever according to claim 1, wherein a dosage of the medicament is in a range from 10 to 80 mg/kg.

4. A medicament for preventing and/or treating pain and/or fever, comprising an effective component, the effective component of the medicament comprises at least one selected from a group consisting of:
a derivative, a pharmaceutically acceptable salt, a solvate, a metabolite, a stereoisomer, a tautomer, a polymorph and a drug precursor of carrimycin;
a derivative, a pharmaceutically acceptable salt, a solvate, a metabolite, a stereoisomer, a tautomer, a polymorph and a drug precursor of isovalerylspiramycin III;
a derivative, a pharmaceutically acceptable salt, a solvate, a metabolite, a stereoisomer, a tautomer, a polymorph and a drug precursor of isovalerylspiramycin II; and
a derivative, a pharmaceutically acceptable salt, a solvate, a metabolite, stereoisomer, a tautomer, a polymorph and a drug precursor of isovalerylspiramycin I.

5. A composite product for preventing and/or treating pain and/or fever, comprising a first drug, and an effective component of the first drug comprises one of carrimycin, isovalerylspiramycin III, isovalerylspiramycin II and isovalerylspiramycin I;
or a combination of two or three of isovalerylspiramycin I, isovalerylspiramycin II and isovalerylspiramycin III.

6. The composite product according to claim 5, wherein the composite product further comprises a second drug, and the second drug comprises at least one of medicaments for preventing and/or treating pain and/or fever; the medicaments for preventing and/or treating pain and/or fever comprise narcotic analgesics, antipyretic analgesics, antibiotics and antiviral drugs.

7. A composite product for preventing and/or treating pain and/or fever, comprising a first drug, an effective component of the first drug comprises at least one selected from a group consisting of:
a derivative, a pharmaceutically acceptable salt, a solvate, a metabolite, a stereoisomer, a tautomer, a polymorph and a drug precursor of carrimycin;
a derivative, a pharmaceutically acceptable salt, a solvate, a metabolite, a stereoisomer, a tautomer, a polymorph and a drug precursor of isovalerylspiramycin III;
a derivative, a pharmaceutically acceptable salt, a solvate, a metabolite, a stereoisomer, a tautomer, a polymorph and a drug precursor of isovalerylspiramycin II; and
a derivative, a pharmaceutically acceptable salt, a solvate, a metabolite, a stereoisomer, a tautomer, a polymorph and a drug precursor of isovalerylspiramycin I.

8. Use of the medicament according to any one of claims 1-3, the medicament according to claim 4, the composite product according to any one of claims 5-6 or the composite product according to claim 7 in preventing and/or treating pain and/or fever.

9. The use according to claim 8, wherein the pain comprises cancer pain.

10. Use of the medicament according to any one of claims 1-3, the medicament according to claim 4, the composite product according to any one of claims 5-6 or the composite product according to claim 7 in reducing or preventing synthesis of a prostaglandin, inhibiting release of a substance P, inhibiting reuptake of a serotonin and a norepinephrine, and inhibiting the activity of a cyclooxygenase.
